(19) Europäisches Patentamt
European Patent Office
Office européen des brevets

(11) Publication number: **0 473 065 A2**

## (12) EUROPEAN PATENT APPLICATION

(21) Application number: **91114072.1**

(22) Date of filing: **22.08.91**

(51) Int. Cl.5: **G01N 33/569**, G01N 33/576, //G01N33/543

(30) Priority: **29.08.90 US 574821**

(43) Date of publication of application: **04.03.92 Bulletin 92/10**

(84) Designated Contracting States: **AT BE CH DE DK ES FR GB GR IT LI NL SE**

(71) Applicant: **ABBOTT LABORATORIES One Abbott Park Road Abbott Park, Illinois 60064-3500(US)**

(72) Inventor: **Brown, William E. III 820 Bedford Lane Libertyville, Illinois 60048(US)**

(74) Representative: **Modiano, Guido et al MODIANO, JOSIF, PISANTY & STAUB Modiano & Associati Via Meravigli, 16 I-20123 Milano(IT)**

(54) Simultaneous assay for detecting two or more analytes.

(57) An assay to simultaneously detect the presence of one or more analytes in a test sample. The analytes are captured on different solid phases, and the presence of one or more analyte is determined by detecting a signal generated by analyte-specific indicator reagents.

EP 0 473 065 A2

## BACKGROUND OF THE INVENTION

This invention relates generally to immunoassays, and more particularly, relates to an immunoassay and products for the simultaneous detection of one or more analytes in a test sample.

Various procedures, devices and reagents have been employed in assays to determine the presence of substances of interest or clinical significance which may be present in a test sample. Such substances commonly are termed "analytes" and can include antibodies, antigens, drugs, hormones, etc.

The detection of more than one analyte in a test sample usually involves the separate detection of each analyte in a separate assay. Such detection methods have been preferred since they allow for stringent quality assurance determinations to be performed for each analyte to be tested.

Advances in medicine have brought a recognition of new markers for many diseases and clinical conditions, along with the demand for clinical tests for these markers. Laboratories are faced with the problem of providing increasing amounts of tests in a timely manner while attempting to keep costs down. For example, the testing requirements of blood banks have increased dramatically due to the addition of Human T-Leukemia Virus Type 1 (HTLV-1), Human Immunodeficiency Virus-1 (HIV-1) and Hepatitis C Virus (HCV) to the panel of agents tested in these laboratories on donor blood for the presence of or exposure to these agents.

One possible solution to reducing the laboratory workload brought about as a result of these requirements is to find ways to combine assays. However, combining assays without compromising their individual performance standards is difficult and more importantly, the problems involved in manufacturing and quality control can be insurmountable.

Several investigators have developed or have attempted to develop assays to simultaneously detect more than one analyte in a test sample. Such an assay would be advantageous since the time involved in detecting more than one analyte in the test sample would diminish considerably, and the cost of each assay would be lowered since less technical time, reagents, and equipment would be required to perform such an assay.

For example, U.S. Patent No. 4,315,907 to Fridlender et al. teaches a heterogeneous specific binding assay system wherein separation of bound-species from a free-species form of the labeled reagent occurs.

U.S. Patent No. 4,378,344 and EP 027008 to Zahradnik et al. teach a solid phase device for determining the presence of each analyte comprising a receptacle and an insert wherein the presence of each analyte is determined by the claimed assay method.

Great Britain Patent No. 2188418 teaches an assay tray assembly having reaction wells each with openings in the top surface from which a projection is extended and wherein the inner surface of each well sidewall and the outer surface of each projection may be incubated simultaneously for detecting two or more specific substances present in a specimen which has been introduced into the reaction wells.

The critical factors which have been identified to the development of simultaneous assays are that the two assays to be performed simultaneously must have the same sample volumes, identical incubation times and identical cut-off calculations. Such a simultaneous assay also should be capable of being separately quality controlled for each analyte, both at the manufacturer and at the laboratory using the assay, to ensure the sensitivity, specificity and reproducibility of the immunoassay.

It therefore would be advantageous to provide an assay wherein the presence of more than one analyte could be simultaneously detected, yet each separate analyte to be detected could be individually quality controlled due to the separate solid phases provided in the system. Such an assay would be an improvement over other known assays since the simultaneous determinations of the presence of either one or more analytes would be performed in one well, separation of solid phase components would not be required, and the assay could be quality controlled for individual analytes which were to be detected in the simultaneous assay.

## SUMMARY OF THE INVENTION

This invention provides an assay to simultaneously detect the presence of one or more analytes which may be present in a test sample. In an embodiment, the assay comprises (a) simultaneously contacting the test sample with (i) a capture reagent for a first analyte, said capture reagent comprising a first binding member specific for a first analyte attached to a first solid phase, and (ii) a capture reagent for a second analyte, said capture reagent comprising a first binding member for a second analyte attached to a second solid phase, thereby forming a mixture; (b) incubating the mixture for a time and under conditions sufficient to form capture reagent/first analyte and capture reagent/second analyte complexes; (c) contacting the formed complexes with (i) an indicator reagent comprising a member of a binding pair specific for the first

2

analyte labelled with a signal generating compound, and (ii) an indicator reagent comprising a member of a binding pair specific for the second analyte labeled with a signal generating compound, thereby forming a second mixture; (d) incubating said second mixture for a time and under conditions sufficient to form (i) capture reagent/first analyte/indicator reagent complexes, and (ii) capture reagent/second analyte/indicator reagent complexes; and (e) determining the presence of one or more analytes in the test sample by detecting a signal generated in connection with the complexes formed on either or both solid phases as an indication of the presence of one or more analytes in the test sample.

In another embodiment, the assay comprises (a) simultaneously contacting the test sample with (i) a capture reagent for a first analyte, said capture reagent comprising a first binding member specific for a first analyte attached to a first solid phase, (ii) a capture reagent for a second analyte, said capture reagent comprising a first binding member specific for a second analyte attached to a second solid phase, (iii) an indicator reagent, said indicator reagent comprising a member of a binding pair specific for the first analyte and labelled with a signal generating compound, thereby forming a mixture; (b) incubating the mixture for a time and under conditions sufficient to form capture reagent/first analyte/indicator reagent complexes and capture reagent/second analyte complexes; (c) contacting the formed complexes with an indicator reagent comprising a member of a binding pair specific for the second analyte labelled with a signal generating compound, thereby forming a second mixture; (d) incubating said second mixture for a time and under conditions sufficient to form capture reagent/second analyte/indicator reagent complexes; and (e) determining the presence of one or more analytes in the test sample by detecting a signal generated in connection with the complexes formed on either or both solid phases as an indication of the presence of one or more analytes in the test sample.

## DETAILED DESCRIPTION OF THE INVENTION

An assay for the simultaneous detection of one or more analytes in a test sample is provided. The assay preferably is performed as an immunoassay, although the present invention is not limited to immunoreactive assays. Any assay utilizing specific binding members can be performed. A "specific binding member," as used herein, is a member of a specific binding pair. That is, two different molecules where one of the molecules through chemical or physical means specifically binds to the second molecule. Therefore, in addition to antigen and antibody specific binding pairs of common immunoassays, other specific binding pairs can include biotin and avidin, carbohydrates and lectins, complementary nucleotide sequences, effector and receptor molecules, cofactors and enzymes, enzyme inhibitors and enzymes, and the like. Furthermore, specific binding pairs can include members that are analogs of the original specific binding member, for example, an analyte-analog. Immunoreactive specific binding members include antigens, antigen fragments; antibodies and antibody fragments, both monoclonal and polyclonal; and complexes thereof, including those formed by recombinant DNA methods.

"Analyte," as used herein, is the substance to be detected which may be present in the test sample. The analyte can be any substance for which there exists a naturally occurring specific binding member (such as, an antibody), or for which a specific binding member can be prepared. Thus, an analyte is a substance that can bind to one or more specific binding members in an assay. "Analyte" also includes any antigenic substances, haptens, antibodies, and combinations thereof. As a member of a specific binding pair, the analyte can be detected by means of naturally occurring specific binding partners (pairs) such as the use of intrinsic factor protein in the capture and/or indicator reagents for the determination of vitamin $B_{12}$, or the use of a lectin in the capture and/or indicator reagents for the determination of a carbohydrate. The analyte can include a protein, a peptide, an amino acid, a hormone, a steroid, a vitamin, a drug including those administered for therapeutic purposes as well as those administered for illicit purposes, a bacterium, a virus, and metabolites of or antibodies to any of the above substances.

The test sample can be a biological fluid such as serum, plasma, cerebrospinal fluid, urine, and the like. The test sample also can be a culture fluid supernatant, or a suspension of cells. It is contemplated that nonbiological fluid samples can be utilized, depending on the types of assays desired to be performed.

The indicator reagent comprises a label conjugated to a specific binding member of each analyte. Each indicator reagent produces a detectable signal at a level relative to the amount of the analyte in the test sample. In a preferred embodiment, each indicator reagent, while comprising a specific binding member of a different analyte, is conjugated to the same signal generating compound (label), which is capable of generating a detectable signal. In general, the indicator reagent is detected or measured after it is captured on the solid phase material. In the present invention, the total signal generated by the indicator reagent(s) indicates the presence of one or more of the analytes in the test sample. It is contemplated that different signal generating compounds can be utilized in the practice of the present invention. Thus, for example, different fluorescent compounds could be utilized as the signal generating compounds, one for each indicator reagent, and detection could be determined by reading at different wavelengths.

In addition to being either an antigen or an antibody member of a specific binding pair, the specific binding member of the indicator reagent can be a member of any specific binding pair, including either biotin or avidin, a carbohydrate or a lectin, a complementary nucleotide sequence, an effector or a receptor molecule, an enzyme cofactor or an enzyme, an enzyme inhibitor or an enzyme, and the like. An immunoreactive specific binding member can be an antibody, an antigen, or an antibody/antigen complex that is capable of binding either to the analyte as in a sandwich assay, to the capture reagent as in a competitive assay, or to the ancillary specific binding member as in an indirect assay. If an antibody is used, it can be a monoclonal antibody, a polyclonal antibody, an antibody fragment, a recombinant antibody, a mixture thereof, or a mixture of an antibody and other specific binding members. The details of the preparation of such antibodies and their suitability for use as specific binding members are well known to those in the art.

The signal generating compound (label) of the indicator reagent is capable of generating a measurable signal detectable by external means. The various signal generating compounds (labels) contemplated include chromagens, catalysts such as enzymes, luminescent compounds such as fluorescein and rhodamine, chemiluminescent compounds, radioactive elements, and direct visual labels. The selection of a particular label is not critical, but it will be capable of producing a signal either by itself or in conjunction with one or more additional substances. A variety of different indicator reagents can be formed by varying either the label or the specific binding member.

The capture reagents of the present invention comprise a specific binding member for each of the analytes of interest which are attached to separate and different solid phases. This attachment can be achieved, for example, by coating the specific binding member onto the solid phases by absorption or covalent coupling. Coating methods, and other known means of attachment, are known to those in the art.

The specific binding member of the capture reagent can be any molecule capable of specifically binding with another molecule. The specific binding member of the capture reagent can be an immunoreactive compound such as an antibody, antigen, or antibody/antigen complex. If an antibody is used, it can be a monoclonal antibody, a polyclonal antibody, an antibody fragment, a recombinant antibody, a mixture thereof, or a mixture of an antibody and other specific binding members.

The solid phase materials can be selected by one of ordinary skill in the art. Thus, wells, beads, and the like, can be utilized. Preferably, polystyrene wells and polystyrene beads are utilized. It is contemplated that both solid phases be present during the quantitation of signal, thus eliminating the need to separate solid phases for detection of signal. A capture reagent for a first analyte is immobilized on the first solid phase, and a capture reagent for a second analyte is immobilized on a second solid phase. The second solid phase preferably is of a different form than the first solid phase. Thus, for example, if the first solid phase selected is a polysytrene well, then the second solid phase utilized in the assay is a polystyrene bead.

In the practice of one embodiment of the present invention, a test sample suspected of containing any of the analytes of interest is simultaneously contacted with a first solid phase to which a first specific binding member of a first analyte is attached, and a second solid phase to which a first specific binding member of a second analyte has been attached, thereby forming a mixture. The specific binding members serve as capture reagents to bind the analyte(s) to the solid phases. If the specific binding member is an immunoreactant, it can be an antibody, antigen, or complex thereof, specific for each analyte of interest. If the specific binding member is an antibody, it can be a monoclonal or polyclonal antibody, an antibody fragment, a recombinant antibody, as well as a mixture thereof, or a mixture of an antibody and other specific binding members. This mixture is incubated for a time and under conditions suffcient for a binding reaction to occur and which incubation results in the formation of capture reagent/first analyte complexes of the first analyte if it is present in the test sample, and/or the formation of capture reagent/second analyte complexes of the second analyte if it is present in the test sample.

Then, an indicator reagent for each analyte is contacted with the complexes. The indicator reagent for the first analyte comprises a specific binding member of the first analyte of interest which has been labelled with a signal generating compound. The indicator reagent for the second analyte comprises a specific binding member of the second analyte of interest which has been labelled with the same signal generating compound as the indicator reagent for the first analyte, thereby forming a second mixture. This second mixture is incubated for a time and under conditions sufficient to form capture reagent/first analyte/indicator reagent complexes and/or capture reagent/second analyte/indicator reagent complexes. The presence of either analyte is determined by detecting the signal generated in connection with the complexes formed on either or both solid phases as an indication of the presence of one or more analytes in the test sample. If the indicator employs an enzyme as the signal generating compound (label), then the signal can be detected visually or measured spectrophotometrically. Or, the label can be detected by the measurement of fluorescence, chemiluminescence, radioactive energy emission, etc., depending on the label used to generate the signal.

In another embodiment of the present invention, a test sample suspected of containing any of the analytes of interest is simultaneously contacted with a first solid phase to which a first specific binding member of a first analyte is attached, a solid phase to which a first specific binding member of a second analyte has been attached, and an indicator reagent for the first analyte comprising a specific binding member for the first analyte labeled with a signal generating compound to form a mixture. The specific binding members serve as capture reagents to bind the analyte(s) to the solid phases. If the specific binding member is an immunoreactant, it can be an antibody, antigen, or complex thereof, specific for each analyte of interest. If the specific binding member is an antibody, it can be a monoclonal or polyclonal antibody, an antibody fragment, a recombinant antibody, as well as mixture thereof, or a mixture of an antibody and other specific binding members. The indicator reagent comprises a specific binding member of the first analyte of interest which has been labelled with a signal generating compound. This mixture is incubated for a time and under conditions sufficient for a binding reaction to occur and which incubation results in the formation of capture reagent/first analyte/indicator reagent complexes of the first analyte and/or capture reagent/second analyte complexes of the second analyte, if either or both the first or second analyte are present in the test sample.

Then, a second indicator reagent comprising a specific binding member of the second analyte of interest and labelled with the same signal generating compound as the indicator reagent for the first analyte, is brought into contact with the so-formed complexes, and this second mixture is incubated for a time and under conditions sufficient to form capture reagent/second analyte/indicator reagent complexes. The presence of either analyte is determined by detecting the signal generated in connection with the complexes formed on either or both solid phases as an indication of the presence of the first analyte and/or the second analyte in the test sample. If the indicator employs an enzyme as the signal generating compound (label), then the signal can be detected visually or measured spectrophotometrically. Or, the label can be detected by the measurement of fluorescence, chemiluminescence, radioactive energy emission, etc., depending on the label used.

Positive and negative controls can be included in the assay of the present invention to ensure reliable results. A blank bead and blank well, to which no capture reagent has been attached, can be utilized as the negative reagent control. Positive controls can include a positive control for each analyte which is tested separately, and a combined positive control wherein the presence of all analytes to be detected in the assay are determined.

Critical factors identified while developing the assay of the present invention included reconciling different amounts of test sample which were required to be used in different assays for different analytes, different incubation times and different cut-off calculations. These factors are summarized in Table 1, wherein "HBsAg" designates Hepatitis B Surface (Coat or Envelope) Antigen and "Anti-HBc" designates anti-Hepatitis B Core Antibody.

TABLE 1

| CURRENT IMMUNOASSAY TESTS* | SPECIMEN VOLUME (ul) | INCUBATION TIMES (minutes) | CUT-OFF CALC. |
|---|---|---|---|
| HBsAg | 150-200 ul | 75/30 | N.C.** + .025 |
| Anti-HBc | 100 ul | 120/30 | 0.4N.C. + 0.6P.C.*** |
| HIV-1 (Current) | 1 ul/400 ul | 60/120/30 | N.C. + .15P.C. |
| HTLV-1 | 5 ul/200 ul | 30/30/30 | 4.5 x N.C. |
| HCV | 5 ul/200 ul | 60/30/30 | N.C. + 0.25 P.C. |

*Based on tests developed and marketed by Abbott Laboratories, Abbott Park, IL.
**N.C. = Negative Control;
***P.C. = Positive Control

It was discovered that a standard volume of test sample could be used in the assay of the present invention to test for one or more analytes.

For example, the traditional sample volume utilized in the assay for HBsAg is ≥ 150 ul, and the sample volume used in the assay for HIV-1 antibody (Ab) has been only 1 to 10 ul. This lower sample volume for HIV-1 was due to the use of an antihuman conjugate in the assay. It has been discovered that the HIV sample volume can be increased to ≥ 150 ul by utilizing a novel assay format which uses a specific antigen-coated solid phase and a specific antigen conjugate. Further, the cut-off calculation for HBsAg has been Negative Control (N.C.) + 0.025, while the cut-off calculation for HIV Ab has been N.C. + 0.10. It was discovered that the cutoff for HBsAg could be increased to N.C. + 0.10 by a dynamic incubation by shaking the reaction tray. This shaking motion, when used at 800 to 1000 rpms, increased the assay kinetics such that the cut-off could be increased to N.C. + 0.10 without loss of sensitivity. It also was discovered that the individual assay incubation times for HBsAg, which were 75 minutes and 30 minutes, could be combined into a common protocol of 90 minutes/30 minutes/30 minutes without loss of test performance.

The manufacturing and quality control problems were resolved by keeping the solid phase for each assay separate in the reaction well. In an embodiment, for example, the solid phase chosen for HBsAg was the inner wall of the reaction well itself, while the solid phase chosen for HIV-1 was a polystyrene bead. By providing separate solid phases, the coating of the solid phases was optimized independently for each analyte and more importantly, each solid phase was capable of being separately quality controlled. When an enzyme is used as the signal-generating compound, color development is detected by reading absorbance values in a spectrophotometer reader with the solid phase bead in the reaction well. Color development (the signal generated by the signal generating compound) comes from either the reaction well or the bead, or both. This assay format thus is characterized as a simultaneous test rather than a combination test, since none of the immunochemical reactions are combined on the same solid phase. This assay format therefore does not identify specific analyte reactions, such as reactivity of the test sample only to HIV-1 or reactivity of the test sample only to HBsAg. Due to the low prevalence of these positive specimens in blood bank populations, however, the one or two true positive specimens per several hundred specimens which are predicted to be present in this population can be identified for further testing. This assay format thus accelerates testing and doubles efficiency and throughput for each laboratory scientist.

The present invention will now be described by way of examples, which are meant to illustrate, but not to limit, the scope of the invention.

**EXAMPLES**

Example 1: Tray Coating Procedure for HCV Antigen

A recombinant HCV antigen was diluted to 133 ng/ml in coating buffer (Phosphate Buffered Saline [PBS], 0.01M, pH 7.2) and mixed thoroughly for a maximum of five (5) minutes at ambient room temperature (22 to 25⁰C). 300 ul of this diluted antigen solution was added to each well of a polysytrene tray. The tray then was incubated dynamically by shaking on an orbital shaker for at least about one (1) hour at ambient room temperature (20 +/- 5⁰C). The diluted antigen solution then was aspirated, 300 ul of a wash buffer (containing PBS, 0.01M, pH 7.2, and 0.05% polysorbate-20) was added to each well of the tray, and the tray was incubated dynamically on an orbital shaker for at least about one (1) hour at ambient room temperature. The wash buffer was aspirated and 400 ul of an overcoat buffer (comprising Tris-Phosphate Buffered Saline with EDTA, EGTA and sucrose as preservatives, 10% Triton X-100® detergent [polyoxyethylene ether, available from Sigma Chemical Corp., St. Louis, MO], 10% calf serum, and 20% goat serum) was added to each well of the tray. The tray then was incubated at 40˚ C for 30 minutes. Following this incubation, the overcoat buffer was removed from each well and the coated trays were dried in a laminar flow hood for approximately 5 hours to approximately 20 hours at ambient room temperature. The trays then were stored at at ambient room temperature until use.

Example 2: Simultaneous Assay for HCV/HTLV-1

The test sample or control was diluted by adding 10 ul of test sample or control to 400 ul of specimen diluent in a test tube. 200 ul of this diluted test sample or diluted control was transferred to a well of a tray prepared by Example 1. One polystyrene bead coated with HTLV-1 antigen (obtained and available from Abbott Laboratories, Abbott Park, IL 60064) was added to each well. The wells were covered, mixed by gently tapping the tray, and incubated at 40 +/- 2⁰C for one (1) hour +/- 5 minutes. The fluid from each bead/well was aspirated, and each bead/well was washed three times using distilled or deionized water. Then, 200 ul of diluted conjugate containing a horseradish peroxidase-labelled goat antibody directed against Human IgG (obtained from Kirkgaard and Perry, Gaithersburg, MD 20879) was added to each bead/well. Each bead/well was covered and mixed by gently tapping the tray. The bead/wells were incubated at 40 +/- 2⁰C for 30 minutes +/- 2 minutes. The fluid from each bead/well was aspirated, and each bead/well was washed three (3) times using distilled or deionized water. Then, 300 ul of o-Phenylenediamine (OPD) substrate was added to each well/bead. The bead/wells were covered and incubated at ambient room temperature for 30 minutes +/- 2 minutes. 300 ul of 1N sulfuric acid was added to each bead/well to stop the color development. The absorbance of each bead/well was read using the Abbott Commander® Parallel Processing Center (available from Abbott Laboratories, Abbott Park, IL 60064), which spectrophotometrically measured the Optical Density (OD) at 460 nm ($OD_{460}$) for each bead/well. The absorbance reading of each test sample was calculated by establishing a cutoff value and determining whether the test sample was less than the cutoff value, or greater than or equal to the cutoff value. Test samples less than the cutoff value were interpreted as nonreactive for both HCV and HTLV-1, while test samples equal to or greater than the cutoff value were interpreted as reactive to either HCV or HTLV-1, or both. The cutoff thus was calculated as follows: CutOff = (0.25xAvg. $OD_{460}$ of Pos. Control) + Avg.$OD_{460}$ of Neg. Control.

Example 3: Reproducibility of HCV/HTLV-1 Simultaneous Assay

A negative control comprising recalcified pooled human plasma which previously tested as negative for HBsAg, anti-HCV, anti-HTLV-1, and anti-HIV (by federally licensed, commercially manufactured tests available from Abbott Laboratories, Abbott Park, IL 60064) and a positive control comprising a recalcified pooled human plasma which had tested positive for antibody to HCV, were tested according to the procedure of Example 2 on two different days. Five (5) separate assay runs were performed on each day. Intra-assay and inter-assay variability was determined from the absorbance data obtained. The data are reported in Table 2.

## TABLE 2

| | DAY 1 | | DAY 2 | |
|---|---|---|---|---|
| | N.C. | P.C. | N.C. | P.C. |
| | 0.21 | 1.852 | 0.273 | 1.805 |
| | 0.277 | 1.753 | 0.273 | 1.859 |
| | 0.258 | 1.953 | 0.294 | 1.971 |
| | 0.273 | 1.974 | 0.326 | 1.946 |
| | 0.263 | 1.889 | 0.332 | 1.783 |
| INTRA-ASSAY | | | | |
| AVG. | 0.256 | 1.884 | 0.3 | 1.873 |
| % cv | 10.5% | 8.8% | 9.4% | 8.3% |

| INTER-ASSAY | N.C. | P.C. |
|---|---|---|
| AVG. | 0.272 | 1.879 |
| %cv | 11.3% | 4.3% |

Example 4: Comparison of Individual Tests and the Present Invention

The assay of the present invention was compared to individual tests for the presence of HCV and HTLV-1 by a side-by-side comparison testing of these methods. Test samples were assayed individually for HCV and HTLV-1 as described hereinbelow, and these results were compared to the results obtained by the assay of the present invention. A 1/4" bead coated with HTLV-1 antigen (obtained and available from Abbott Laboratories, Abbott Park, IL) and a 1/4" bead coated with two (2) purified recombinant proteins representing several distinct epitopes on HCV (obtained and available from Abbott Laboratories, Abbott Park, IL 60064) were used separately to determine the presence of these markers, and the bead/well assay of Example 2 was employed to determine the presence of HCV/HTLV-1 in the same test samples. The individual beads were tested as follows, following the product insert for the Abbott Commander® Parallel Processing Center:

10 ul of each control or specimen was dispensed into the bottom of an individual test tube. 400 ul of a specimen diluent was added to each tube, and the tube was mixed. Then, 200 ul of each diluted specimen or control was transferred into a well of a polystyrene tray. One bead coated with the appropriate antigen was added to each well. The wells were covered with a cover seal, and the trays were gently tapped to cover the beads and to remove any air bubbles trapped in the well. The wells were incubated at 40 +/- 2°C for one (1) hour +/- five (5) minutes. Following this incubation, the seals were removed from the wells, the liquid was aspirated from each well, and each bead was washed with a total of 12 to 18 ml of distilled or deionized water. Then, 200 ul of a diluted conjugate specific for the assayed analyte was pipetted into each well containing a bead. A cover seal was applied to the wells of the tray and any air bubbles present were removed by gently tapping the tray. The wells were incubated at 40 +/- 2°C for 30 +/- 2 minutes. Following this incubation the cover seals were removed, the liquid present in each well was aspirated and the wells were washed with a total of 12 to 18 ml of distilled or deionized water. Then, 300 ul of freshly prepared OPD solution was added to a first row of a blank tray which contained blanking beads and then the OPD solution was added into each well containing a coated bead. The wells were covered with a cover seal and incubated at ambient room temperature for 30 +/- 2 minutes. The color reaction was stopped by adding 300 ul of 1N sulfuric acid to each well. The absorbance reading ($OD_{460}$) of the blanks, controls and specimens was measured. The cutoff for HCV was calculated as follows: (0.25 x Avg. $O.D._{460}$ of N.C). A sample was determined to be non-reactive to HCV if it was less than the cutoff. A sample was determined to be reactive to HCV when the $O.D._{460}$ was greater than or equal to the cutoff. The cutoff for HTLV-1 was calculated as follows: 4.5 x Avg. $O.D._{460}$ of N.C. A sample was determined to be reactive to HTLV-1 when its absorbance reading was less than the cutoff. A sample was determined to be reactive to HTLV-1 when its absorbance reading was greater than or equal to the cutoff.

Twenty-nine (29) patients suspected of being HCV reactive were tested individually for HCV, HTLV-1 as described herein and also for HCV/HTLV-1 by the assay of the present invention as described herein. The $OD_{460}$ readings for the negative and positive controls, as well as the sample/cutoff ratios (S/CO) for the 29 patients, are summarized in Table 3. The cutoffs were calculated as follows:

HTLV-1 Cutoff: 4.4 x $NC_x$;

HCV Cutoff: .25 x PC + $NC_x$;

HTLV-1/HCV Cutoff: .25 x PC + $NC_x$.

## TABLE 3

| SAMPLE | HTLV-1 | Interp. | HCV | Interp. | HTLV-1/HCV | Interp. |
|---|---|---|---|---|---|---|
| $NC_x$(O.D.) | 0.267 | - | 0.109 | - | 0.2562 | - |
| $PC_x$(O.D.) | 1.401 | + | 1.042 | + | 1.8842 | + |
| 1 (S/CO)** | 0.107 | - | 2.49 | + | 1.59 | + |
| 2 | 0.149 | - | 0.59 | - | 0.41 | - |
| 3 | 0.117 | - | 7.01 | + | 3.03 | + |
| 4 | 0.106 | - | 8.37 | + | 3.03 | + |
| 5 | 0.106 | - | 0.66 | - | 0.60 | - |
| 6 | 0.126 | - | 8.12 | + | 3.03 | + |
| 7 | 0.088 | - | 4.32 | + | 3.03 | + |
| 8 | 0.158 | - | 0.75 | - | 0.34 | - |
| 9 | 0.130 | - | 8.37 | + | 3.03 | + |
| 10 | 0.243 | - | 8.37 | + | 3.03 | + |
| 11 | 0.101 | - | 0.44 | - | 0.32 | - |
| 12 | 0.112 | - | 0.79 | - | 0.48 | - |
| 13 | 0.147 | - | 0.65 | - | 0.31 | - |
| 14 | 0.141 | - | 0.55 | - | 0.34 | - |
| 15 | 0.331 | - | 1.54 | +/-* | 0.65 | - |
| 16 | 0.150 | - | 0.59 | - | 0.31 | - |
| 17 | 0.080 | - | 7.54 | + | 3.03 | + |
| 18 | 0.140 | - | 6.37 | + | 3.03 | + |
| 19 | 0.162 | - | 6.77 | + | 3.03 | + |
| 20 | 0.119 | - | 8.37 | + | 3.03 | + |
| 21 | 0.067 | - | 6.36 | + | 3.03 | + |
| 22 | 0.135 | - | 4.78 | + | 3.03 | + |
| 23 | 0.125 | - | 8.37 | + | 3.03 | + |
| 24 | 0.074 | - | 8.37 | + | 3.03 | + |
| 25 | 0.111 | - | 8.37 | + | 3.03 | + |
| 26 | 0.076 | - | 8.37 | + | 3.03 | + |
| 27 | 0.081 | - | 8.37 | + | 3.03 | + |
| 28 | 0.135 | - | 2.34 | + | 1.61 | + |
| 29 | 0.125 | - | 6.76 | + | 3.03 | + |

* (-) on retest; the S/CO is provided for samples 1 to 29.

| TOTAL REACTIVE | 0/29 | | 20/29 | | 20/29 | |
|---|---|---|---|---|---|---|

Fifteen (15) patients confirmed by Western Blot as positive for HTLV-1 were tested in the same manner described above. The data, reported in S/CO ratios, are shown in Table 4.

TABLE 4

| SAMPLE | HTLV-1 | Interp. | HCV | Interp. | HTLV-1/HCV | Interp. |
|--------|--------|---------|------|---------|------------|---------|
| 1 | 1.228 | + | 7.99 | + | 3.03 | + |
| 2 | 1.527 | + | 8.37 | + | 3.03 | + |
| 3 | 1.666 | + | 8.37 | + | 3.03 | + |
| 4 | 1.828 | + | 8.37 | + | 3.03 | + |
| 5 | 0.703 | +/- | 8.37 | + | 3.03 | + |
| 6 | 1.828 | + | 0.84 | - | 3.03 | + |
| 7 | 1.227 | + | 8.37 | + | 3.03 | + |
| 8 | 1.410 | + | 0.42 | - | 2.21 | + |
| 9 | 1.828 | + | 8.37 | + | 3.03 | + |
| 10 | 1.828 | + | 0.45 | - | 3.03 | + |
| 11 | 1.828 | + | 8.37 | + | 3.03 | + |
| 12 | 1.426 | + | 0.38 | - | 2.14 | - |
| 13 | 1.828 | + | 0.54 | - | 3.03 | + |
| 14 | 1.828 | + | 0.59 | - | 3.03 | + |
| 15 | 1.828 | + | 0.62 | - | 3.03 | + |
| TOTAL REACTIVE | 14/15 | | 8/15 | | | 15/15 |

The data from Tables 3 and 4 demonstrate the overall sensitivity for both HCV and HTLV-1 employing the assay of the present invention. The results obtained from the assay of the present invention were equal to the results obtained with the individual assays utilizing 1/4" beads for separate determinations.

Fifty (50) fresh plasma or serum test samples obtained from a blood bank were tested by the procedures described hereinabove. The data demonstrated that no test sample reacted with HTLV-1 when tested by the individual 1/4" bead assay. Four (4) of these 50 test samples tested "+/-" (inconclusive) for HCV when tested by the individual 1/4" bead assay. On retest, these four test samples tested nonreactive. When these 50 test samples were tested by the assay of the present invention, all 50 tested nonreactive.

Similarly, another fifty (50) normal serum samples were tested by the methods herein described. It was found that one (1) sample tested inconclusive with the individual 1/4" bead assay for HTLV-1, which sample was nonreactive on retest. Also, two (2) samples were inconclusive when tested with the individual 1/4" bead assay for HCV. On retest, these two samples tested nonreactive. All 50 test samples tested nonreactive by the assay of the present invention.

Example 5: Tray Coating Procedure for anti-HBs Antibody

200 ul of a 15 mM EDAC/0.2M propylamine solution was added to each well of each tray to be coated. Then, 200 ul of a 15 ug/ml solution of anti-HBs monoclonal antibody was added to each well of each tray. Each tray was rotated at 200 rpms for 70 minutes at ambient room temperature. The coating solution then was aspirated, and 800 ul of a PBS/0.1% Tween® (polyoxyethylenesorbitan, available from Sigma Chem. Co., St.Louis, MO) wash solutions was added to each well. Each well was incubated for 30 minutes at 40°C. Following incubation, this wash solution was aspirated from each well, and 500 ul of a 0.1M citrate/3% BSA/5% sucrose overcoat solution was added to each well. The wells were incubated for 60 minutes at ambient room temperature. Following this incubation, the overcoat solution was aspirated from each well. The wells then were dried at 40°C for 30 minutes and stored at ambient room temperature until use.

Example 6: Simultaneous Assay for HBsAg and HIV-1/2 Antibody

150 ul of test sample and 50 ul of HIV specimen diluent/HBsAg enzyme conjugate (which contained goat anti-HBs:Horseradish Peroxidase [HRPO], JM103 [E. coli lysates from lysed cells and E. coli protein] and CKS lysates [cytidiane monophosphate-KDO synthetase] were added to assay wells prepared by Example 5. A bead coated with an antigen preparation containing antigen of HIV-1 and HIV-2 ("HIV-1/2")

10

(obtained and available from Abbott Laboratories, Abbott Park, IL 60064) then was added to the well. This mixture was incubated for 90 minutes at $40^0$C, aspirated, and washed twice with distilled water. Any HBsAg analyte, if present in the test specimen, formed an antibody:antigen:antibody-enzyme complex on the reaction well. Then, 200 ul of a HIV antigen-enzyme conjugate was added to the well. This mixture was incubated for 30 minutes and washed as previously described. Any HIV-1 Ab analyte, if present in the test specimen, formed an antigen:antibody:antigen-enzyme conjugate on the bead. Next, 300 ul of OPD was added and color was allowed to develop for 30 minutes by incubating the bead/well at ambient room temperature. The reaction(s) were quenched by adding 300 ul of 1N sulfuric acid, and absorbance readings were taken by measuring the $OD_{460}$. The test was processed and read on the Abbott Commander® Parallel Processing Center (available from Abbott Laboratories, Abbott Park, Illinois 60064).

EXAMPLE 7: Testing of Normal Citrate Plasma Population to HBsAg

The procedure described in Example 6 was used to test 958 normal citrated plasma test samples. Of the 958 test samples, four (4) samples (0.42%) initially tested reactive using the method of the present invention. Upon retest with the present method, two (2) of the initial four (4) reactive test samples tested reactive (0.21%). Repeat reactive test samples then were tested by the Western Blot confirmatory procedure and found to be negative for HIV antibody. Testing with the Auszyme® IV antibody neutralization procedure (Abbott Laboratories, Abbott Park, IL 60064) was performed on all test samples which were repeatably reactive with the method of the present invention, and the two repeatably reactive test samples were confirmed as positive.

EXAMPLE 8: Testing of Normal Citrate Plasma Population for HBsAg

The procedure described in Example 7 was performed using a different lot of wells prepared by Example 5 and beads coated with an antigen preparation containing HIV-1 and HIV-2 antigen (available from Abbott Laboratories. Abbott Park, IL 60064). One thousand ninety-eight (1398) normal citrated plasma test samples were assayed according to the method described in Example 6. Initially, it was found that six (6) test samples (0.43%) of these 1398 samples tested reactive by the method of the present invention. Of these 6 samples, 3 were repeat reactive (0.21%) after retesting with the present method was performed. Repeat reactive test samples then were tested by the Western Blot confirmatory procedure for HIV and were negative; two(2) of these three (3) test samples (0.14%) were found to be confirmed positive for HBsAg by testing with the Auzyme® IV antibody neutralization procedure (available from Abbott Laboratories, Abbott Park, IL 60064).

EXAMPLE 9: Testing of HIV-1 Sensitivity Panel

The procedure described in Example 7 was performed using an HIV-1 seroconversion panel. The results obtained were compared to the results obtained by Enzyme Immunoassay (EIA) using the currently available and federally approved test available from Abbott Laboratories, Abbott Park, IL 60064. These results are reported in Table 5 below.

## TABLE 5

| Panel Member Number | Present Invention S/CO | Interp. | EIA S/CO | Interp. |
|---|---|---|---|---|
| 1 | .35 | - | .14 | - |
| 2 | .63 | - | .11 | - |
| 3 | 1.80 | + | .16 | - |
| 4 | 2.64 | + | 1.08 | + |
| 5 | 1.18 | + | 1.13 | + |
| 6 | 1.22 | + | 1.70 | + |
| 7 | 2.84 | + | 1.70 | + |
| 8 | 4.32 | + | 4.74 | + |

The data from Table 5 indicate that the method of the present invention had greater sensitivity than the current EIA method since it detected a positive reaction in panel member number 3, which reaction was not detected in that panel member with the current EIA test. It is hypothesized that the present invention may be more sensitive due to the ability of the present invention to additionally detect IgM.

EXAMPLE 10: HBsAg Sensitivity Panel

The procedure of Example 7 was performed using positive and negative controls, and known quantities of HBsAg antigens and antibodies. The results Auzyme® IV test (Abbott Laboratories, Abbott Park, IL 60064) was utilized. These data are summarized in Table 6.

## TABLE 6

| Specimen Tested | Auzyme® IV ($A_{460}$) | Present Method ($A_{460}$) |
|---|---|---|
| Negative Control | 0.016 | 0.034 |
| Positive Control | 0.625 | 1.136 |
| AD* (.92 ng/ml) | 0.045 | 0.175 |
| AD (.51 ng/ml) | 0.026 | 0.116 |
| AD (.31 ng/ml) | 0.025 | 0.087 |
| AY* (1.11 ng/ml) | 0.048 | 0.232 |
| AY (.50 ng/ml) | 0.026 | 0.124 |
| AY (.30 ng/ml) | 0.019 | 0.081 |
| Cutoff | 0.041 | 0.134 |
| AD sensitivity | 0.85 | 0.63 |
| AY sensitivity | 0.94 | 0.59 |

* AD and AY are subtype designations for the HBsAg.

The data from Table 6 indicate that the claimed method is more sensitive for the AD and AY subtypes of HBsAg than the Auzyme® IV test. This increase in sensitivity represents another advantage of the claimed invention over that of currently available licensed methodologies.

It will be apparent that various changes and modifications can be made from the details of the invention as described herein without departing from the spirit and scope of the attached claims.

**Claims**

1. An assay to simultaneously detect the presence of one or more analytes which may be present in a test sample, comprising:
   a. simultaneously contacting the test sample with
      i. a capture reagent for a first analyte comprising a first binding member specific for a first analyte attached to a first solid phase,
      ii. a capture reagent for a second analyte comprising a first binding member specific for a second analyte attached to a second solid phase, thereby forming a mixture;
   b. incubating the mixture for a time and under conditions sufficient to form capture reagent/first analyte complexes and/or capture reagent/second analyte complexes;
   c. contacting the formed complexes with
      i. an indicator reagent for the first analyte comprising a member of a binding pair specific for the first analyte labelled with a signal generating compound, and
      ii. an indicator reagent for the second analyte comprising a member of a binding pair specific for the second analyte labelled with a signal generating compound, thereby forming a second mixture;
   d. incubating the second mixture for a time and under conditions sufficient to form capture reagent/first analyte/indicator reagent complexes and/or capture reagent/second analyte/indicator reagent complexes; and
   e. determining the presence of one or more analytes in the test sample by detecting a signal generated in connection with the complexes formed on either or both solid phases as an indication of the presence of one or more analytes in the test sample.

2. The assay of claim 1 wherein the signal generating compound of the indicator reagent for the first analyte and the signal generating compound of the indicator reagent for the second compound is selected from the group consisting of enzymes, luminescent compounds, chemiluminescent compounds and radioactive elements.

3. The assay of claim 1 wherein the first analyte is anti-HCV antibody and the first binding member specific for the first analyte is HCV antigen.

4. The assay of claim 1 wherein the second analyte is HTLV-1 antigen and the first binding member specific for the second analyte is HTLV-1 antibody.

5. An assay to simultaneously detect the presence of one or more analytes which may be present in a test sample, comprising:
   a. simultaneously contacting the test sample with:
      i. a capture reagent for a first analyte comprising a first binding member specific for a first analyte attached to a first solid phase,
      ii. a capture reagent for a second analyte comprising a first binding member specific for a second analyte attached to a second solid phase.
      iii. an indicator reagent for the first analyte comprising a member of a binding pair specific for the first analyte labelled with a signal generating compound, thereby forming a mixture;
   b. incubating the mixture for a time and under conditions sufficient to form capture reagent/first analyte/indicator reagent complexes and capture reagent/second analyte complexes;
   c. contacting the formed complexes with an indicator for the second analyte comprising a member of a binding pair specific for the second analyte labelled with a signal generating compound, thereby forming a second mixture;
   d. incubating the second mixture for a time and under conditions sufficient to form capture reagent/second analyte/indicator reagent complexes; and
   e. determining the presence of the first analyte and/or the second analyte in the test sample by detecting a signal generated in connection with the complexes formed on either or both solid phases as an indication of the presence of the first analyte and/or the second analyte in the test sample.

6. The assay of claim 5 wherein the signal generating compound for the first analyte and the signal generating compound for the second analyte is selected from the group consisting of enzymes, luminescent compounds, chemiluminescent compounds and radioactive elements.

7. The assay of claim 5 wherein the first analyte is Hepatitis B surface antigen and the first binding member specific for the first analyte is anti-Hepatitis B Surface antibody.

8. The assay of claim 5 wherein the second analyte is HIV antibody and the first binding member specific for the second analyte is HIV antigen.

9. A kit for simultaneously detecting one or more analytes in a test sample, comprising:
   a. a capture reagent for a first analyte comprising a first binding member specific for a first analyte attached to a first solid surface;
   b. a capture reagent for a second analyte comprising a second binding member specific for a second analyte attached to a second solid surface;
   c. an indicator reagent for the first analyte comprising a member of a binding pair specific for the first analyte labelled with a signal generating compound;
   d. an indicator reagent for the second analyte comprising a member of a binding pair specific for the second analyte labelled with a signal generating compound.

10. The kit of claim 9 wherein said signal generating compound for the indicator reagent for the first analyte and the signal generating compound for the indicator reagent for the second analyte is selected from the group consisting of enzymes, luminescent compounds, chemiluminescent compounds and radioactive elements.